# FASCICULE DE BREVET EUROPEEN

(11) **EP 2 237 769 B1**
(45) Date de publication et mention de la délivrance du brevet: **31.10.2012**
(21) Numéro de dépôt: 08872768.0
(22) Date de dépôt: 17.12.2008
(51) Int. Cl.: A61K 9/107, A61K 31/165, A61P 31/04, A23K 1/00

(54) **COMPOSITION LIQUIDE AUTOÉMULSIONNABLE DE FLORFENICOL DESTINÉE A ÊTRE INCORPORÉE À L'EAU DE BOISSON DES ANIMAUX D'ÉLEVAGE**
SELBSTEMULGIERBARE FLÜSSIGE FLORFENICOLZUSAMMENSETZUNG ALS ZUSATZ FÜR DAS TRINKWASSER VON NUTZTIEREN
SELF-EMULSIFIABLE LIQUID FLORFENICOL COMPOSITION INTENDED TO BE INCORPORATED INTO THE DRINKING WATER OF LIVESTOCK ANIMALS

(30) Priorité: 26.12.2007 FR 0709106
(43) Date de publication de la demande: 13.10.2010
(73) Titulaire: VIRBAC S.A., F-06516 Carros (FR)
(72) Inventeur: DERRIEU, Guy, F-06800 Cagnes sur Mer (FR); RAYNIER, Bernard, F-06200 Nice (FR)
(74) Mandataire: Macquet, Christophe
(86) Numéro de dépôt international: PCT/FR2008/001757
(87) Numéro de publication internationale: WO 2009/106714

(56) Documents cités:
- WO-A-99/56727
- WO-A-02/053131
- WO-A-2004/014341
- ANON: "glycosyl" [On line] 2 novembre 2007 (2007-11-02). Extrait de l'Internet: URL: http://en.wikipedia.org/wiki/Glycosyl> [extrait le 2008-09-26] XP002497563

## Description

La présente invention concerne une composition liquide auto-émulsionnable comprenant le principe actif Florfénicol, destinée à être incorporée à l'eau de boisson des animaux d'élevage et, notamment, à l'eau de boisson des porcs, des volailles, des ovins, des caprins et des bovins. Elle concerne plus particulièrement une telle composition liquide auto-émulsionnable de Florfénicol à usage vétérinaire.

Les animaux d'élevage sont sujets à toutes sortes de maladies dont la plupart sont contagieuses. Le risque est donc important, lorsque des symptômes apparaissent chez un animal, de les voir se propager rapidement à l'ensemble du groupe avec lequel il vit, et aux autres groupes proches. Aussi, il apparaît souvent nécessaire, dès l'apparition d'une pathologie chez un animal, de traiter rapidement, en même temps que celui-ci, tous les animaux abrités sur le même site d'élevage, en leur administrant le même traitement curatif ou préventif.

La voie d'administration la plus adéquate pour traiter un grand nombre d'animaux, outre l'incorporation d'une médication dans un aliment sec ou humide, est l'incorporation d'une telle médication dans l'eau de boisson. En effet, les animaux malades continuent de boire pour compenser l'hyperthermie et la déshydratation, même si leur appétit est réduit. De plus, par rapport à l'aliment sec ou humide, l'emploi de l'eau de boisson permet une flexibilité et une continuité de mise en oeuvre, avec modulation de dose et association de traitement. L'emploi de l'eau de boisson assure également une meilleure homogénéité du traitement et une meilleure régularité de dosage. Enfin, l'eau de boisson garantit également, par rapport à l'aliment sec ou humide, une rapidité d'intervention et d'assimilation des traitements.

Les élevages intensifs actuels sont, pour la plupart, équipés de circuits de circulation de l'eau de boisson. Ces circuits alimentent des auges adaptées aux animaux de taille relativement importante tels que des porcs ou veaux, ou alors, des petites pipettes plus spécialement adaptées aux petits animaux tels que la volaille.

Pour l'administration du Florfénicol dans l'eau de boisson, on détermine, dans une première étape, la concentration de consigne ou dose d'emploi du Florfénicol dans l'eau de boisson, en tenant compte de la posologie prescrite, des tables de croissance ainsi que de la consommation d'eau moyenne des animaux à traiter. Puis, la concentration de consigne ayant ainsi été déterminée, il convient d'ajouter, dans le circuit de circulation, la quantité de composition liquide concentrée en principe actif permettant d'obtenir cette concentration de consigne dans les auges ou dans les pipettes où viennent se désaltérer les animaux à traiter. En règle générale, l'administration du principe actif passe par une étape de préparation d'une dilution intermédiaire aqueuse dans un bac de dilution intermédiaire des traitements du circuit de circulation. Cette dilution intermédiaire présente une concentration en actif inférieure à la concentration en principe actif de la composition liquide concentrée et supérieure à la concentration de consigne.

L'utilisation d'une composition liquide concentrée en principe actif présente de nombreux avantages. Elle est parfaitement adaptable aux besoins de ces animaux. Cependant, sa mise au point implique la résolution de nombreux problèmes et est, par conséquent, particulièrement difficile.

En particulier, le Florfénicol utilisé régulièrement dans le traitement des animaux d'élevage, présente une faible solubilité, de l'ordre d'environ 1,32 g/l, dans l'eau à température ambiante. Cela pose donc un problème pour la mise au point de la composition liquide concentrée mais aussi au moment de la dilution intermédiaire. Ainsi, dans la dilution intermédiaire, la concentration en Florfénicol est généralement bien supérieure à son seuil de solubilité. Ceci provoque notamment une hétérogénéité dans cette dilution intermédiaire, due à une sédimentation du principe actif, entraînant une mauvaise distribution du Florfénicol dans les auges et dans les pipettes, mais aussi, un colmatage des différents éléments du circuit de circulation qui peut se produire au niveau du tube d'alimentation d'une pompe doseuse, au niveau des organes de commande permettant l'activation automatique du circuit de circulation d'eau, et au niveau des pipettes, lorsque le traitement est destiné à des petits animaux, tels que les volailles.

Il est donc particulièrement important que la composition liquide concentrée en Florfénicol soit parfaitement homogène et stable, à tous les stades de dilution, et qu'elle permette d'obtenir une dissolution complète de ce principe actif lors de la dilution finale dans l'eau de boisson se trouvant au niveau des auges et des pipettes où viennent se désaltérer les animaux à traiter.

Pour le Florfénicol, analogue structural du thiamphénicol qui est un antibiotique synthétique à large spectre, principalement bactériostatique, il existe déjà des compositions concentrées comme le produit NUFLOR^{®} commercialisé par la société Schering-Plough. Ces compositions, contenant une concentration de Florfénicol de 2,3% en poids par rapport au volume total de la composition (p/v), présentent toutefois certains des inconvénients mentionnés ci-dessus.

Ainsi, les compositions existantes à ce jour ne fournissent pas une assez bonne solubilité des particules de principe actif dans les auges et les pipettes des animaux lors de la dilution de la préparation dans l'eau de boisson, avec cristallisation plus ou moins conséquente selon la dilution visée. En outre, de telles compositions doivent impérativement être conservées à une température inférieure à 25°C, et présentent une conservation très limitée après dilution dans l'eau de boisson.

Pour résoudre ce problème, une des solutions apportées, divulguée dans la demande internationale WO 02/49609, consiste à obtenir une suspension aqueuse de principe actif dont la taille moyenne des particules est inférieure à 20 microns. Cela nécessite d'obtenir au préalable un principe actif micronisé avant l'incorporation de celui-ci dans la suspension. Cette solution, compte tenu de la nature des principes actifs, qui présentent une très faible vitesse de dissolution, ne donne pas entière satisfaction dans le processus de dilution. En effet, il apparaît dans le bac de dilution ainsi que dans les auges et les pipettes, un phénomène de sédimentation qui crée un gradient de concentration dans l'eau de boisson. De plus, il est connu de l'homme de l'art qu'il est très difficile d'obtenir, sauf par des techniques complexes et coûteuses, des particules ayant une taille moyenne inférieure à 5 microns. Cet inconvénient rend l'application de cette solution plus complexe.

Une solution à ce problème posé consiste en la mise au point d'une composition liquide auto-émulsionnable comprenant un principe actif et susceptible d'être soumise à une dilution aqueuse ayant une teneur en actif qui peut être supérieure à la limite de solubilité de l'actif dans l'eau, tout en respectant :
- l'intégrité de l'actif et l'homogénéité de la distribution de l'actif dans le diluat correspondant ;
- la stabilité physique du diluat correspondant en inhibant ou limitant toute sédimentation ;
- l'aptitude de pouvoir transférer le diluat correspondant dans l'eau de boisson des animaux d'élevage en utilisant les procédés classiquement disponibles ;
et en assurant une solubilité rapide et totale du principe actif dans l'eau de boisson.

La demande internationale PCT WO2004/014341 décrit une composition pharmaceutique pour administration orale comprenant une microémulsion contenant du florfénicol comme principe actif et dans laquelle la microémulsion est obtenue en ajoutant une huile et un agent tensio-actif à une solution de florfénicol dans un milieu de dispersion hydrophile ou amphiphile. La demande internationale PCT WO02/053131 décrit, quant à elle, des compositions pharmaceutiques renfermant un principe actif très lipophile, permettant d'accroître la disponibilité de principes actifs insolubles dans les solvants désignés par l'appellation MIDDS® (Micellar Improved Drug Delivery Solution). Enfin, la demande internationale PCT WO99/56727 décrit des compositions capables de former une émulsion ou une microémulsion huile dans eau après dilution dans une solution aqueuse.

La présente invention s'est par conséquent fixée pour but de pouvoir disposer d'une composition contenant au moins le principe actif Florfénicol, apte au traitement d'animaux d'élevage intensif en particulier des porcs, des volailles, des ovins, des caprins et des bovins, se présentant sous la forme d'une composition liquide auto-émulsionnable stable susceptible d'être introduite dans les circuits de circulation de l'eau de boisson des animaux dont disposent de nombreux élevages. Cette composition liquide auto-émulsionnable évoluera lors de la dilution intermédiaire vers une émulsion homogène et stable pendant au moins 24 heures, et permettra d'obtenir rapidement une solubilité totale du Florfénicol dans l'eau de boisson se retrouvant dans les auges et dans les pipettes où viennent se désaltérer les animaux à traiter. La demanderesse s'est aperçue que, lors de la dilution intermédiaire aqueuse, la composition de l'invention forme une émulsion. On entend par émulsion selon l'invention, un liquide formé par dispersion complète et uniforme de très petites particules qui lui confèrent les propriétés de l'état colloïdal stable. L'état colloïdal stable se rapporte à une substance composée de très petites particules qui sont dispersées de manière homogène dans un matériau liquide, particules résistants à la floculation et à l'agrégation dans le temps.

Ainsi, la solution proposée selon l'invention a pour premier objet une composition liquide auto-émulsionnable destinée à être incorporée à l'eau de boisson des animaux d'élevage comprenant au moins :
- entre 1 et 12 % du principe actif Florfénicol en poids par rapport au volume total de la composition (P/v);
- au moins 50% en poids par rapport au volume total de la composition (p/v) de PEG-8 caprylic/capric glycérides, et
- au moins 5% en poids par rapport au volume total de la composition (p/v) d'un agent tensioactif selon la revendication 1.

Elle a pour second objet un procédé d'incorporation d'une telle composition dans de l'eau de boisson des animaux d'élevage, caractérisé en ce qu'il comporte les étapes suivantes de :
- dilution de ladite composition auto-émulsionnable dans l'eau d'un bac de dilution de manière à former une émulsion ; et
- d'incorporation de ladite émulsion, à la dose souhaitée, dans l'eau de boisson des animaux d'élevage.

Enfin, elle a pour troisième objet l'utilisation d'une composition telle que définie ci-dessus :
- comme médicament à usage vétérinaire ;
- pour la préparation d'une émulsion apte à être diluée dans l'eau de boisson pour traiter les animaux d'élevage ; ou
- pour la préparation d'un médicament administrable par voie orale apte à être dilué dans l'eau de boisson pour prévenir ou traiter les maladies des animaux d'élevage.

La composition auto-émulsionnable selon l'invention présente donc la particularité de former, une fois incorporée dans l'eau du réseau général de distribution lors de la dilution intermédiaire, une émulsion permettant d'assurer une bonne stabilité et une homogénéité de la dilution intermédiaire et de permettre l'obtention rapide d'une solubilité totale du principe actif dans l'eau de boisson présent dans les auges et dans les pipettes où viennent se désaltérer les animaux à traiter.

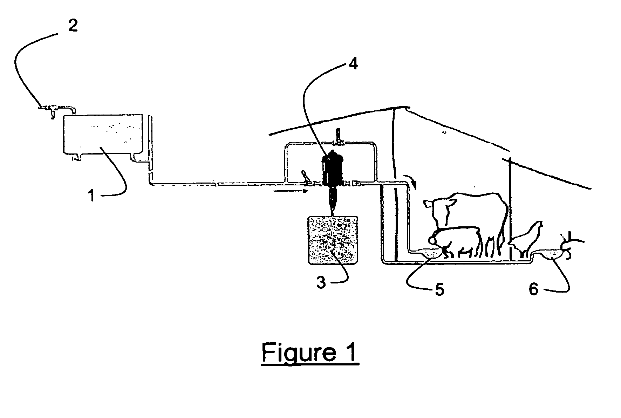

L'invention sera mieux comprise à la lecture de la description non limitative qui va suivre et des dessins annexés, dans lesquels les figures 1, 2 et 3 présentent différentes variantes de circuits de circulation de l'eau de boisson pour animaux d'élevage.

La composition selon l'invention est destinée à être incorporée à l'eau de boisson des animaux d'élevage. Cette eau de boisson provient généralement d'un réseau général de distribution d'eau. Elle parcourt ensuite un circuit de circulation. Ce circuit débouche sur des auges ou pipettes. Les animaux d'élevage s'abreuvent dans ces auges ou pipettes.

Dans le mode de réalisation présenté à la figure 1, le circuit de circulation est relié au réseau 2 de distribution d'eau. Il comporte, immédiatement en aval de ce réseau, un réservoir gravitaire 1. Ce réservoir 1 est susceptible de contenir un grand volume d'eau, suffisant pour combler les besoins en eau potable des animaux pendant une durée déterminée, de préférence une journée. Il est relié, par une conduite, à un bac 3 de dilution des traitements. Ce bac 3 est muni d'une pompe doseuse 4. L'eau extraite du réseau de distribution 2 s'écoule dans le réservoir gravitaire 1. Pour le traitement des animaux, l'eau contenue dans ce bac 1 est amenée jusqu'à la pompe 4. Celle-ci est actionnée. Une quantité calculée du diluat de traitement contenu dans le bac 3 est alors incorporée à l'eau circulante dans le circuit. L'eau du circuit, incorporant le diluat à la dose d'emploi de l'actif contenu dans la composition selon l'invention, est finalement déversée dans les auges 5 et/ou pipettes 6 dans lesquelles s'abreuvent les animaux. En règle générale, la circulation d'eau dans le circuit est activée automatiquement par des organes de commande associés en particulier à des sondes de niveau placées dans les auges 5 ou pipettes 6, ou encore, à des détecteurs de présence d'un animal.

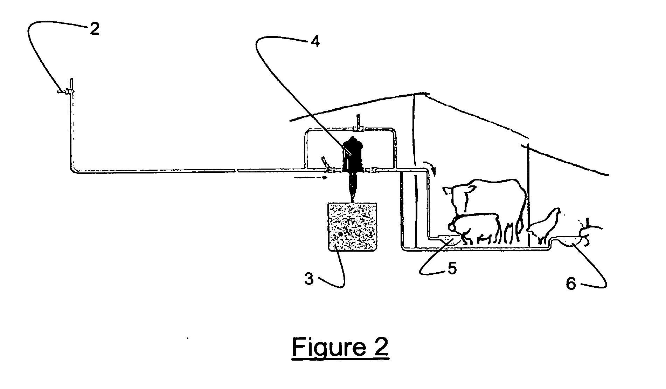

Dans le mode de réalisation présenté à la figure 2, le circuit de circulation est de même relié au réseau 2 de distribution d'eau. Il ne comporte cependant pas de réservoir gravitaire mais uniquement un bac 3 de dilution des traitements muni d'une pompe doseuse 4 actionnée, à la demande, pour le traitement des animaux.

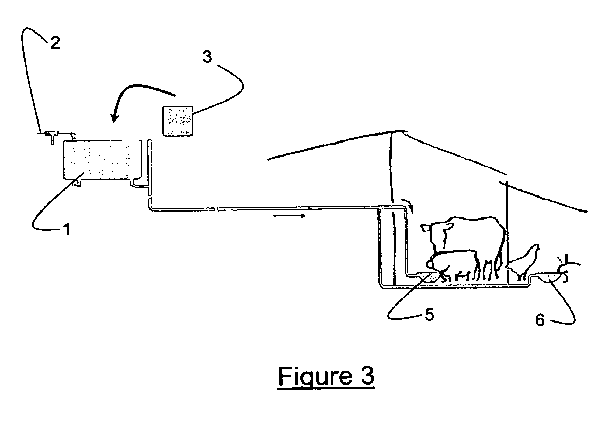

Enfin, dans le mode de réalisation présenté à la figure 3, le circuit de circulation de l'eau de boisson pour animaux d'élevage ne présente pas de pompe doseuse. Le bac de dilution 3 est séparé du circuit. La dilution de la composition selon l'invention s'effectue dans ce bac séparé, qui est directement déversé dans le réservoir gravitaire, à la dose d'emploi de l'actif contenu dans la composition.

L'invention consiste donc notamment à avoir sélectionné un mélange de glycérides polyglycolysés saturés en C8-C10 et présentant une HLB (balance hydrophile-lipophile) inférieure à 16 particulière qui, combiné avec des agents tensioactifs spécifiques, forme une émulsion en présence de l'eau apportée par le réseau général de distribution.

A titre d'exemple pour le PEG-8 caprylic/capric glycérides, on peut citer le produit commercialisé par la société Gattefosse sous le nom « LABRASOL^{™} » (Caprylocaproyl Macrogolglycerides (Polyoxylglycerides)).

Selon l'invention, par agent tensioactif (TA), on entend un composé chimique possédant deux groupements, le premier polaire ou ionique, qui présente une grande affinité pour l'eau, le second qui contient une chaîne aliphatique plus ou moins longue et est hydrophobe. Ces composés chimiques sont destinés à provoquer la formation de micelles.

L'agent tensioactif (TA) est choisi parmi les esters lipophiles du polyglycerol et les esters du propylène glycol engageant des acides gras en C8-C18. On retiendra préférentiellement les esters oléiques du polyglycérol, du type de celui commercialisé par la société Gattefosse sous le nom « PLUROL OLEIQUE^{™} » (Polyglycérol oléate), les esters lauriques du propylène glycol, tel que celui commercialisé par la société Gattefosse sous la marque déposée « LAUROGYCOL^{™} » (Propylène glycol monolaurate), et les esters capryliques du propylène glycol, tel que celui commercialisé par la société Gattefosse sous la marque déposée « CAPRYOL 90^{™} » (Propylène glycol caprylic acid mono ester ou Propylene glycol mono caprylate).

Selon l'invention, le rapport entre le mélange de glycérides polyglycolysés saturés en C8-C10 et présentant une HLB (balance hydrophile-lipophile) inférieure à 16 et le tensioactif est compris entre 3 et 7 et de préférence voisin de 5.

Selon un mode de réalisation avantageux de l'invention, la composition comprend :
- entre 1 et 12 % de principe actif Florfénicol en poids par rapport au volume total de la composition (p/v), préférentiellement entre 1,5 et 6% (poids/volume) de Florfénicol et encore plus préférentiellement entre 2 et 4 % p/v de Florfénicol ;
- au moins 50% en poids par rapport au volume total de la composition (p/v) de mélange de glycérides polyglycolysés saturés en C8-C10 et présentant une HLB (balance hydrophile-lipophile) inférieure à 16 ; et
- au moins 5% en poids par rapport au volume total de la composition (p/v) d'un agent tensioactif selon la revendication 1.

Selon une forme de réalisation préférée de l'invention, l'agent tensioactif représente de 5 à 40% en poids par rapport au volume total de la composition (p/v).

Le PEG-8 caprylic/capric glycérides mentionné ci-dessus sert à ajuster au volume final requis la composition liquide auto-émulsionnable. Le pourcentage poids/volume du mélange de glycérides dépend de la nature (donc de la densité) de celui-ci.

La composition selon l'invention peut en outre contenir un ou plusieurs tensioactifs non ioniques additionnels, miscibles à l'eau et physiologiquement acceptables, choisis parmi :
- Les polyéthers d'huile de ricin hydrogénée et d'oxyde d'éthylène, soit plus précisément le PEG-40 huile de ricin hydrogénée ou le PEG-60 huile de ricin hydrogénée, produits connus respectivement sous les noms commerciaux de Cremophor RH40^{™} et Cremophor RH60^{™}. On retiendra de préférence le Cremophor RH60^{™};
- Les monoesters de polyoxyéthylène-sorbitan, identifiés également sous le nom générique de polysorbates, monoesters avec des acides gras tels que laurique, palmitique, stéarique, oléique. On retiendra particulièrement le PEG-20 sorbitan monolaurate, le PEG-20 sorbitan monopalmitate, le PEG-20 sorbitan monostéarate, le PEG-20 sorbitan monooléate, produits connus respectivement sous les noms commerciaux Tween 20^{™}, Tween 40^{™}, Tween 60^{™} et Tween 80^{™}. On retiendra de préférence le Tween 80^{™};

- Les esters d'acides gras et d'oligoéthylèneglycols, avec en particulier le PEG-20 stéarate et le PEG-50 stéarate, produits connus respectivement sous les noms commerciaux Simulsol M49^{™} et Simulsol M53^{™}; et
- Les éthers d'alcools gras et d'oligoéthylèneglycols, avec en particulier le PEG-20 hexadécanol, le PEG-23 dodécanol, le PEG-20 octadécanol et le PEG-20 oléyl alcool, produits connus respectivement sous les noms commerciaux Simulsol 58^{™}, Simulsol P4^{™}, Simulsol 78^{™} et Simulsol 98^{™}.

La composition selon l'invention peut également intégrer des solvants tels que notamment les éthers de glycol, plus particulièrement l'éther éthylique du diéthylène glycol, les polyéthylène-glycols, les dérivés pyrrolidine-2-one N-substitués (C1-C4) ou non ; de préférence l'éther éthylique ou le diéthylène glycol (« Transcutol^{™} »), la pyrrolidine-2-one (« Soluphor P^{™} ») et la N-methyl -2-pyrrolidone.

Enfin, la composition selon l'invention peut également intégrer des auxiliaires de formulation tels que les antioxydants, les conservateurs, les édulcorants ou autres appétants régulièrement utilisés et admis pour ce type de composition.

Bien entendu, l'homme du métier veillera à choisir le ou les éventuels composants à ajouter à ces compositions et leurs quantités respectives de telle manière que les propriétés avantageuses attachées intrinsèquement à la présente invention ne soient pas ou substantiellement pas altérées par l'addition envisagée. Par exemple, Lorsque l'on passe à une concentration de Florfénicol d'environ 2,3% (poids/volume), il est préférable d'ajouter à la composition un tensioactif non ionique tel que mentionné ci-dessus et lorsque l'on vise une concentration supérieure à 4,5% (poids/volume), soit 6 à 10% (poids/volume) de Florfénicol pour 100 ml, il est préférable d'ajouter un solvant tel que mentionné ci-dessus.

Selon un premier mode préféré de réalisation de l'invention, la composition liquide auto-émulsionnable est une solution stable comprenant :
- de 4 à 5 g de Florfénicol pour 100 ml;
- de 14 à 18 g de Lauroglycol^{™} pour 100 ml ; et
- de 82 à 90 g de Labrasol^{™} pour 100 ml.

Selon un deuxième mode préféré de réalisation de l'invention, la composition liquide auto-émulsionnable est une solution stable comprenant :
- de 4 à 5 g de Florfénicol pour 100 ml ;
- de 14 à 18 g de Plurololéique pour 100 ml ; et
- de 82 à 90 g de Labrasol^{™} pour 100 ml.

Selon un troisième mode préféré de réalisation de l'invention, la composition liquide auto-émulsionnable est une solution stable comprenant :
- de 2 à 3 g de Florfénicol pour 100 ml ;
- de 11 à 13 g de Lauroglycol^{™} pour 100 ml ;
- de 21 à 32 g de Polysorbate 80 pour 100 ml ; et
- de 55 à 75 g de Labrasol^{™} pour 100 ml

Les compositions selon l'invention sont utilisées comme médicament à usage vétérinaire.

Ainsi, l'invention concerne également l'utilisation de la composition selon l'invention pour la préparation d'une émulsion apte à être diluée dans l'eau de boisson pour traiter les animaux d'élevage. Elle se rapporte également à l'utilisation de la composition selon l'invention pour la préparation d'un médicament apte à être dilué dans l'eau de boisson pour traiter les animaux d'élevage dans les indications usuelles du Florfénicol, à savoir par exemple le traitement préventif et curatif des infections de l'appareil respiratoire dues à *Mannheimia haemolytica, Pasteurella multocida et Histophilus somni* chez les bovins et les traitement des infections respiratoires dues à *Actinbacillus pleuropneumoniae* et *Pasteurella multocida* chez les porcins.

Les exemples qui suivent viennent illustrer la présente invention, avec plus précisément des exemples concrets de compositions selon l'invention.

### Exemple 1

### Compositions selon l'invention

### Composition 1 :

| Nom | Quantité |
|---|---|
| Florfénicol | 2,3 g |
| Lauroglycol | 12,6 g |
| Polysorbate 80 | 28,0 g |
| Labrasol | qsp 100 ml |

### Composition 2

| Nom | Quantité |
|---|---|
| Florfénicol | 2, 3 g |
| Plurol Oléïque | 12,4 g |
| Polysorbate 80 | 30,0 g |
| Labrasol | qsp 100 ml |

### Composition 3

| Nom | Quantité |
|---|---|
| Florfénicol | 4,5 g |
| Lauroglycol | 17,0 g |
| Labrasol | qsp 100 ml |

### Composition 4

| Nom | Quantité |
|---|---|
| Florfénicol | 4,5 g |
| Plurol Oléïque | 17,2 g |
| Labrasol | qsp 100 ml |

### Composition 5

| Nom | Quantité |
|---|---|
| Florfénicol | 6,0 g |
| Lauroglycol | 12,4 g |
| Ethyl diglycol | 25,0 g |
| Labrasol | qsp 100 ml |

### Composition 6

| Nom | Quantité |
|---|---|
| Florfénicol | 8,0 g |
| Lauroglycol | 14, 6 g |
| N-methyl-2-pyrrolidone | 20,0 g |
| Labrasol | qsp 100 ml |

### Composition 7

| Nom | Quantité |
|---|---|
| Florfénicol | 10,00 g |
| Lauroglycol | 11, 95 g |
| Pyrrolidine -2-one | 30,00g |
| Labrasol | qsp 100 ml |

### Exemple 2

La composition 1 décrite dans l'exemple 1 a été testée dans le détail pour les dilutions de 1 à 10 g/l, avec des dosages en actif sur 24 heures.

On observe une stabilité du Florfénicol dans le temps pour les dilutions de 1 à 10 g/l, à température ambiante, sur un laps de temps supérieur à 24h. En outre, on n'observe pas de gradient de concentration au niveau des dilutions intermédiaires au repos lorsqu'elles présentent une limpidité.

On retrouve un gradient de concentration lorsque l'opacité notée pour les dilutions entre 1,0 et 3,3 g/l s'accompagne d'un déphasage éventuel, soit à la concentration théorique de 0,2 g/l. Ce gradient n'est pas d'emblée conséquent : le recouvrement est de 98,9% à 102,4% au bout d'1h30, puis tend vers un équilibre, avec des résultats particulièrement proches pour 15h30 et 24h : recouvrement de 94,4% et 94,6% en haut ; de 93,4% et 94,9% au milieu ; et de 116,5% pour les deux temps en bas.

Ainsi, on peut penser qu'une légère agitation, telle que par exemple le flux engendré par la pompe d'aspiration, pourrait assurer l'homogénéité de l'ensemble.

On observe également une homogénéité de la dilution à 2 g/l à température ambiante après 15h lorsqu'une légère agitation est assurée avant le prélèvement, quel que soit le type de prélèvement, sans ou avec filtration (0,22 ou 1 micron). Ce résultat confirme que l'actif apparaît sous une forme colloïdale.

On a également évalué les compositions 5, 6 et 7 de l'exemple 1 en dilution aqueuse aux concentrations 2, 5 et 10 g/l. Ces compositions montrent une stabilité physique sur 8 jours et plus à 4°C. Pour la dilution intermédiaire à 2 g/l, on observe un aspect opaque assez léger, stable pendant 24 heures. Pour la dilution intermédiaire à 5 g/l, on observe un aspect opaque plus dense qu'à 2 g/l. Pour la dilution intermédiaire à 10 g/l, on n'observe pas de changements par rapport à la dilution intermédiaire à 5 g/l.

### Exemple 3

Nous allons ici détailler les caractéristiques particulièrement attractives des compositions auto-émulsionnables selon l'invention contenant 2,3% (poids/volume) de Florfénicol.

### a. Etude d'une composition auto-émulsionnable selon l'invention contenant 2,3% (poids/volume) de Florfénicol sur 24 heures.

### COMPOSITION SELON L'INVENTION :

- Florfénicol 23 mg
- Excipients qsp 1 ml

### CARACTERISTIQUES PRODUIT :

### Conservation

### 24 mois

### Posologie et mode d'emploi dans les conditions suivantes :

- Température d'environ 25°C
- Dureté de l'eau inférieure à 275ppm

### Dans le réservoir d'eau :

Pour traiter les porcs de 8 à 200 kg, la concentration en Florfénicol doit être comprise entre 0,10 et 0,17 g/l de boisson.

On ne rencontre aucun obstacle pour les concentrations envisagées. En effet, la composition auto-émulsionnable apparait diluable avec une légère opalescence du milieu. De plus, les concentrations sont en dessous de la solubilité du Florfénicol à savoir 1,32 g/l.

### Pompes doseuses :

Quatre types d'équipement de pompes doseuses sont envisagés : 1%, 2%, 5% et 10%.

### A. Pompe doseuse à 1% :

Pour traiter les porcs de 8 à 200 kg, la concentration en Florfénicol dans le réservoir de la pompe doseuse doit être comprise entre 10 et 16,7 g/l d'eau.

On n'observe aucun problème pour les concentrations souhaitées. En effet, la composition auto-émulsionnable est diluable. De plus, certaines concentrations apparaissent au-delà de la « miscibilité » du Florfénicol à savoir 12 g/l.

### B. Pompe doseuse à 2%

Afin de traiter les porcs de 8 à 200 kg, la concentration en Florfénicol dans le réservoir de la pompe doseuse doit être comprise entre 5 et 8,3 g/l d'eau. On n'observe pas de problème pour les concentrations souhaitées. La composition auto-émulsionnable apparaît diluable.

### C. Pompe doseuse à 5%

Pour traiter les porcs de 8 à 200 kg, la concentration en Florfénicol dans le réservoir de la pompe doseuse doit être comprise entre 2 et 3,3 g/l d'eau.

On observe qu'à une concentration de 2 g/l, le milieu est opaque mais reste homogène. Un déphasage apparaît après 6h. Pour les concentrations supérieures à 2 g/l, la composition auto-émulsionnable apparaît diluable.

### D. Pompe doseuse à 10%

Pour traiter les porcs de 8 à 200 kg, la concentration en Florfénicol dans le réservoir de la pompe doseuse doit être comprise entre 1 et 1,65 g/l d'eau.

De façon, surprenante, on n'observe pas de problème pour les concentrations souhaitées. La composition auto-émulsionnable apparaît diluable, donnant un milieu opaque homogène. De plus, certaines concentrations sont en dessous de la solubilité du Florfénicol à savoir 1,32 g/l. A une concentration de 1,65 g/l, le milieu est opaque homogène, puis, il apparaît un déphasage après 6h.

### Conclusion :

Il est indiqué dans le mode d'emploi du produit « Nuflor 2,3% » commercialisé par la société Schering-Plough que les solutions dont les concentrations en Florfénicol sont comprises entre 1,2 g/l et 12 g/l de Florfénicol précipitent. Compte tenu des éléments présentés ci-dessus, il apparaît qu'au contraire, la composition auto-émulsionnable selon l'invention permet d'obtenir une préparation homogène à ces concentrations de Florfénicol.

Des essais additionnels ont aussi permis de mettre en évidence que la composition selon l'invention pouvait être utilisée à des conditions physiques critiques. Ainsi, des essais à une température de 5°C et en présence d'une eau dure ont été réalisés et sont concluants.

### b. Etude d'une composition auto-émulsionnable selon l'invention contenant 2,3% (poids/volume) de Florfénicol sur 8 à 10 heures dans des conditions physiques critiques.

### COMPOSITION SELON L'INVENTION :

- Florfénicol 23 mg
- Excipients qsp 1 ml

### CARACTERISTIQUES PRODUIT :

### Conservation

### 24 mois

### Posologie et mode d'emploi dans les conditions suivantes :

- Température d'environ 5°C
- Dureté de l'eau comprise entre 370 et 375ppm

### Dans le réservoir d'eau :

Pour traiter les porcs de 8 à 200 kg, la concentration en Florfénicol doit être comprise entre 0,10 et 0,17 g/l de boisson.

On ne rencontre aucun obstacle pour les concentrations envisagées. En effet, la composition auto-émulsionnable apparaît diluable avec une légère opalescence du milieu. De plus, les concentrations sont en dessous de la solubilité du Florfénicol à savoir 1,32 g/l.

### Pompes doseuses :

Quatre types d'équipement de pompes doseuses sont envisagés : 1%, 2%, 5% et 10%.

### A. Pompe doseuse à 1%

Pour traiter les porcs de 8 à 200 kg, la concentration en Florfénicol dans le réservoir de la pompe doseuse doit être comprise entre 10 et 16,7 g/l d'eau.

La composition auto-émulsionnable est diluable.

### B. Pompe doseuse à 2%

Afin de traiter les porcs de 8 à 200 kg, la concentration en Florfénicol dans le réservoir de la pompe doseuse doit être comprise entre 5 et 8,3 g/l d'eau.

On n'observe pas de problème pour les concentrations souhaitées. La composition auto-émulsionnable apparaît diluable.

### C. Pompe doseuse à 5%

Pour traiter les porcs de 8 à 200 kg, la concentration en Florfénicol dans le réservoir de la pompe doseuse doit être comprise entre 2 et 3,3 g/l d'eau.

On observe qu'aux concentrations souhaitées, la composition auto-émulsionnable est diluable, donnant un milieu opaque homogène. Puis il apparaît un déphasage après 6h.

### D. Pompe doseuse à 10%

Pour traiter les porcs de 8 à 200 kg, la concentration en Florfénicol dans le réservoir de la pompe doseuse doit être comprise entre 1 et 1,65 g/l d'eau.

On observe qu'aux concentrations souhaitées, la composition auto-émulsionnable est diluable, donnant un milieu opaque homogène.

### Conclusion :

Ces essais additionnels ont permis de mettre en évidence que la composition selon l'invention pouvait être utilisée à des conditions physiques critiques, c'est-à-dire notamment à une température de 5°C et en présence d'une eau de dureté comprise entre 370 et 375ppm.

En outre, lors de l'emploi de pompes, pour les concentrations comprises entre 1,32 g/l et 12 g/l, il apparaît avantageux de maintenir une agitation douce pour permettre à l'émulsion de rester parfaitement homogène.

## Revendications

1. Composition liquide auto-émulsionnable destinée à être incorporée à l'eau de boisson des animaux d'élevage comprenant au moins :
- entre 1 et 12% du principe actif Florfénicol en poids par rapport au volume total de la composition ;
- au moins 50% en poids par rapport au volume total de la composition (p/v) de PEG-8 caprylic/capric glycérides ; et
- au moins 5% en poids par rapport au volume total de la composition (p/v) d'un agent tensioactif choisi parmi les esters lipophiles du polyglycérol et les esters du propylène glycol engageant des acides gras en C8-C18.

2. Composition selon la revendication 1, **caractérisée en ce que** l'agent tensioactif choisi parmi les esters lipophiles du polyglycérol et les esters du propylène glycol engageant des acides gras en C8-C18 est un ester oléique du polyglycérol, un ester laurique du propylène glycol ou un ester caprylique du propylène glycol.

3. Composition selon l'une des revendications précédentes, **caractérisée en ce que** l'agent tensioactif choisi parmi les esters lipophiles du polyglycérol et les esters du propylène glycol engageant des acides gras en C8-C18, représente de 5 à 40% en poids par rapport au volume total de la composition (p/v).

4. Composition selon l'une des revendications précédentes, **caractérisée en ce qu'**elle comprend en outre un agent tensioactif non ionique choisi parmi les polyéthers d'huile de ricin hydrogénée et d'oxyde d'éthylène, les monoesters de polyoxyéthylène-sorbitan, les esters d'acide gras et d'oligoéthylèneglycols, les éthers d'alcools gras et d'oligoéthylèneglycols.

5. Composition selon l'une des revendications précédentes, **caractérisée en ce qu'**elle comprend en outre un solvant choisi parmi les éthers de glycol, les polyéthylène-glycols, les dérivés pyrrolidine-2-one N-substitués (C1-C4) ou non, la pyrrolidine-2-one et la N-methyl-2-pyrrolidone.

6. Composition selon l'une quelconque des revendications 1 à 5 comme médicament à usage vétérinaire.

7. Composition selon l'une quelconque des revendications 1 à 6, **caractérisée en ce qu'**elle comprend :
- de 4 à 5 g de Florfénicol pour 100 mL ;
- de 14 à 18 g d'un ester laurique du propylène glycol ou d'un ester oléique du polyglycérol pour 100 mL ; et
- de 82 à 90 g de PEG-8 caprylic/capric glycérides pour 100 mL.

8. Composition selon l'une quelconque des revendications 1 à 6, **caractérisée en ce qu'**elle comprend :
- de 2 à 3 g de Florfénicol pour 100 mL ;
- de 11 à 13 g d'un ester laurique du propylène glycol pour 100 mL ;
- de 21 à 32 g de PEG-20 sorbitan monooléate pour 100 mL ; et
- de 55 à 75 g de PEG-8 caprylic/capric glycérides pour 100 mL.

9. Procédé d'incorporation d'une composition selon l'une des revendications 1 à 8, dans de l'eau de boisson des animaux d'élevage, **caractérisé en ce qu'**il comporte les étapes suivantes de :
- dilution de ladite composition auto-émulsionnable dans l'eau d'un bac de dilution (3) de manière à former une émulsion ; et
- d'incorporation de ladite émulsion, à la dose souhaitée, dans l'eau de boisson des animaux d'élevage.

10. Utilisation d'une composition selon l'une quelconque des revendications 1 à 8 pour la préparation d'une émulsion apte à être diluée dans l'eau de boisson pour traiter les animaux d'élevage.

11. Utilisation d'une composition selon l'une quelconque des revendications 1 à 8 pour la préparation d'un médicament administrable par voie orale apte à être dilué dans l'eau de boisson pour prévenir ou traiter les maladies des animaux d'élevage.

## Claims

1. Self-emulsifiable liquid composition intended to be incorporated into the drinking water of livestock animals, comprising at least:
- between 1 and 12% of the active ingredient Florfenicol, by weight relative to the total volume of the composition;
- at least 50% by weight relative to the total volume of the composition (w/v) of PEG-8 caprylic/capric glycerides; and
- at least 5% by weight relative to the total volume of the composition (w/v) of a surfactant selected from lipophilic esters of polyglycerol and esters of propylene glycol comprising C8-C18 fatty acids.

2. The composition according to claim 1, **characterized in that** the surfactant selected from lipophilic esters of polyglycerol and esters of propylene glycol comprising C8-C18 fatty acids is an oleic ester of polyglycerol, a lauric ester of propylene glycol or a caprylic ester of propylene glycol.

3. The composition according to any one of the preceding claims, **characterized in that** the surfactant selected from lipophilic esters of polyglycerol and esters of propylene glycol comprising C8-C18 fatty acids amounts to 5 to 40% by weight relative to the total volume of the composition (w/v).

4. The composition according to any one of the preceding claims, **characterized in that** it further comprises a nonionic surfactant selected from polyethers of hydrogenated castor oil and ethylene oxide, polyoxyethylene sorbitan monoesters, esters of fatty acids and oligoethylene glycols, ethers of fatty alcohols and oligoethylene glycols.

5. The composition according to any one of the preceding claims, **characterized in that** it further comprises a solvent selected from glycol ethers, polyethylene glycols, the pyrrolidine-2-one derivatives, whether N-substituted (C1-C4) or not, pyrrolidine-2-one and N-methyl-2-pyrrolidone.

6. The composition according to any one of claims 1 to 5, as a medication for veterinary use.

7. The composition according to any one of claims 1 to 6, **characterized in that** it comprises:
- from 4 to 5 g of Florfenicol for 100 mL;
- from 14 to 18 g of a lauric ester of propylene glycol or of an oleic ester of polyglycerol for 100 mL; and
- from 82 to 90 g of PEG-8 caprylic/capric glycerides for 100 mL.

8. The composition according to any one of claims 1 to 6, **characterized in that** it comprises:
- from 2 to 3 g of Florfenicol for 100 mL;
- from 11 to 13 g of a lauric ester of propylene glycol for 100 mL;
- from 21 to 32 g of PEG-20 sorbitan monooleate for 100 mL; and
- from 55 to 75 g of PEG-8 caprylic/capric glycerides for 100 mL.

9. A method for incorporating a composition according to any one of claims 1 to 8, into the drinking water of livestock animals, **characterized in that** it comprises the following steps:
- diluting said self-emulsifiable composition in water from a dilution tank (3) so as to form an emulsion; and
- incorporating said emulsion, at the desired dose, into the drinking water of livestock animals.

10. Use of a composition according to any one of claims 1 to 8 for the preparation of an emulsion suitable for dilution into drinking water for treating livestock animals.

11. Use of a composition according to any one of claims 1 to 8 for the preparation of a medication for oral administration suitable for dilution into drinking water for preventing or treating diseases of livestock animals.

## Patentansprüche

1. Eine flüssige, selbst-emulgierbare Zusammensetzung, die dazu bestimmt ist, dem Trinkwasser von Zuchttieren beigemischt zu werden, wobei sie zumindest umfasst:
- zwischen 1 und 12% des Wirkstoffes Florfenicol, bezogen auf das Gesamtvolumen der Zusammensetzung;
- mindestens 50%, bezogen auf das Gesamtvolumen der Zusammensetzung (Gew/V), von PEG-8 Capryl/Capric Glyceride; und
- mindestens 5%, bezogen auf das Gesamtvolumen der Zusammensetzung (Gew/V), eines Tensids, ausgewählt aus lipophilen Estern von Polyglycerin und Estern von Propylenglykol, die C8-C18-Fettsäuren aufweisen.

2. Zusammensetzung nach Anspruch 1, **dadurch gekennzeichnet, dass** das aus den lipophilen Polyglycerin und Estern von Polyglycerin und Estern von Propylenglykol, die C8-C18-Fettsäuren aufweisen, ausgewählte Tensid, ein Öl-Ester von Polyglycerin, ein Laurinsäureester von Propylenglykol, oder ein Capryl Ester von Propylenglykol ist.

3. Zusammensetzung nach einem der vorhergehenden Ansprüche, **dadurch gekennzeichnet, dass** das aus den lipophilen Polyglycerin und Estern von Polyglycerin und Estern von Propylenglykol, die C8-C18-Fettsäuren aufweisen, ausgewählte Tensid, zwischen 5 und 40 Gew.% des Gesamtvolumen der Zusammensetzung beträgt (Gew/V).

4. Zusammensetzung nach einem der vorhergehenden Ansprüche, **dadurch gekennzeichnet, dass** sie außerdem ein nichtionisches Tensid beinhaltet, das aus Polyethern von hydriertem Rizinusöl und Ethylenoxid, Monoestern von Polyoxyethylensorbitan, Estern von Fettsäuren und Oligo-Ethylenglykolen, Ethern aus Fettalkoholen und Oligo-Ethylenglykolen ausgewählt wird.

5. Zusammensetzung nach einem der vorhergehenden Ansprüche, **dadurch gekennzeichnet, dass** sie außerdem ein Lösungsmittel beinhaltet, das aus Glykolethern, Polyethylenglykolen, aus Derivaten von Pyrrolidin-2-one, die N-substituiert (C1-C4) oder nicht substituiert sind, aus Pyrrolidin-2-one und aus N-Methyl-2-Pyrrolidon ausgewählt wird.

6. Zusammensetzung nach einem der Ansprüche 1 bis 5, die als Medikament im veterinären Anwendungsbereich verwendet wird.

7. Zusammensetzung nach einem der Ansprüche 1 bis 6, **dadurch gekennzeichnet, dass** sie umfasst:
- 4 bis 5 g Florfenicol auf 100ml;
- 14 bis 18 g eines Laurinsäureester von Propylenglykol oder eines Öl-Ester von Polyglycerin auf 100ml;
- 82 bis 90 g PEG-8 Capryl/Capric Glyceride auf 100ml.

8. Zusammensetzung nach einem der Ansprüche 1 bis 6, **dadurch gekennzeichnet, dass** sie umfasst:
- 2 bis 3 g Florfenicol auf 100ml;
- 11 bis 13 g eines Laurinsäureester von Propylenglykol auf 100 ml;
- 21 bis 32 g PEG-20 Sorbitanmonooleat auf 100 ml; und
- 55 bis 75 g PEG-8 Capryl/Capric Glyceride auf 100 ml.

9. Verfahren zur Beimischung einer Zusammensetzung nach einem der Ansprüche 1 bis 8 dem Trinkwasser von Zuchttieren beigemischt wird, **dadurch gekennzeichnet, dass** es die folgenden Schritte aufweist:
- Verdünnen der besagten selbst-emulgierbaren Zusammensetzung im Wasser einer Lösungsschale (3), um eine Emulsion zu bilden ; und
- Beimischung der besagten Emulsion in das Trinkwasser der Zuchttiere, in der gewünschten Dosis.

10. Verwendung einer Zusammensetzung nach einem der Ansprüche 1 bis 8 zur Herstellung einer Emulsion, geeignet für die Auflösung in Trinkwasser zur Behandlung von Zuchttieren.

11. Verwendung einer Zusammensetzung nach einem der Ansprüche 1 bis 8 zur Herstellung eines Medikaments zur oralen Einnahme, geeignet zur Auflösung in Trinkwasser, um Krankheiten von Zuchttieren vorzubeugen oder zu behandeln.
